# EUROPÄISCHE PATENTSCHRIFT

(11) **EP 0 241 022 B1**
(45) Veröffentlichungstag und Bekanntmachung des Hinweises auf die Patenterteilung: **13.01.1993**
(21) Anmeldenummer: 87105230.4
(22) Anmeldetag: 08.04.1987
(51) Int. Cl.: C07K 3/08, C12N 9/36, C12N 9/04, C12N 9/64

(54) **Verfahren zur Renaturierung von Proteinen**
Process for the renaturation of proteins
Procédé pour la renaturation de protéines

(30) Priorität: 08.04.1986 DE 3611817
(43) Veröffentlichungstag der Anmeldung: 14.10.1987
(73) Patentinhaber: BOEHRINGER MANNHEIM GMBH, 68298 Mannheim (DE)
(72) Erfinder: Rudolph, Rainer, Dr. rer. nat., D-8400 Regensburg (DE); Fischer, Stephen, Dr. rer. nat., D-8120 Weilheim (DE)
(74) Vertreter: Huber, Bernhard, Dipl.-Chem.

(56) Entgegenhaltungen:
- EP-A- 0 156 354
- WO-A-84/03711
- US-A- 4 216 141
- BIOPHYSICS OF STRUCTURE AND MECHANISM, Band 8, 1982, Seiten 231-256, Springer-Verlag; R. JAENICKE: "Folding and association of proteins"
- Biochemistry 16, 3384-90 (1977)

## Beschreibung

Die Erfindung betrifft ein Verfahren zur Renaturierung von Proteinen.

Unter Renaturierung von Proteinen wird im Rahmen der vorliegenden Erfindung die Herstellung oder Wiederherstellung der normalen biologischen Funktion von Proteinen verstanden, wobei angenommen wird, daß hierbei die natürliche Sekundär-, Tertiär- und gegebenenfalls Quartärstruktur wiederhergestellt wird.

Dem Problem der Renaturierung von Proteinen kommt große Bedeutung zu, da biologisch aktive Proteine vielfach durch die daran vorgenommenen Handlungen, beispielsweise bei ihrer Abtrennung von Begleitstoffen, denaturiert werden und hierbei die interessierende biologische Aktivität ganz oder teilweise einbüßen. Renaturierung ist auch von Bedeutung bei synthetisch oder nach Methoden der Gentechnologie hergestellten Proteinen, da man hierbei in vielen Fällen nur die richtige Primärstruktur, also Aminosäuresequenz, erhält, die angestrebte Ausnutzung der biologischen Aktivität aber voraussetzt, daß eine Renaturierung unter Ausbildung der natürlichen Sekundärstruktur, Tertiärstruktur und gegebenenfalls auch der Quartärstruktur gelingt.

Aus der US-PS 4,530,787 ist es bereits bekannt, synthetische Proteine, welche reduziertes Cystein enthalten, mit Jodosobenzoat unter Wiedergewinnung ihrer biologischen Aktivität zu oxidieren. Selbst wenn diese Methode allgemein anwendbar sein sollte, würde sie doch nur ein Teilproblem der Renaturierung von Proteinen lösen.

G. Zettlmeissl, R. Rudolph und R. Jaenicke, Biochemistry Vol. 18 (1979), Seiten 5567 bis 5575, haben die Renaturierung eines oligomeren Enzyms, nämlich der Lactat-Dehydrogenase, unter Wiedergewinnung ihrer Tertiärstruktur untersucht und dabei festgestellt, daß bei einer solchen Renaturierung zwei konkurrierende Reaktionen ablaufen, nämlich die Renaturierung einerseits und die Bildung von inaktiven Aggregaten andererseits. Für die Erzielung guter Renaturierungsausbeuten mußte dabei darauf geachtet werden, daß eine Grenzkonzentration an Enzymen in der Lösung nicht überschritten wird. Ein allgemeines Verfahren zur Renaturierung von Proteinen läßt sich daraus jedoch nicht herleiten.

Aus WO-A-84/03711 ist es bekannt rekombinantes Methionin-Prochymosin nach Auflösung in Denaturierungspuffer langsam auf das 10- bis 50-fache Volumen durch Zugabe zu gerührtem Verdünnungspuffer zu verdünnen und durch anschließendes Stehenlassen, Herabsetzung des pH-Wertes und erneutes Stehenlassen, Ansäuern und Neutralisation in katalytisch aktives Chymosin zu überführen. Die eigentliche Renaturierung erfolgt hierbei durch Verringerung des pH-Wertes der Lösung. Kritische Bedingungen, die für die Renaturierung wesentlich sind, lassen sich darüberhinaus aus dieser Literaturstelle nicht entnehmen.

Aufgabe der Erfindung ist deshalb die Schaffung eines Verfahrens, welches generell eine Verbesserung der Renaturierung von Proteinen ermöglicht.

Gelöst wird diese Aufgabe erfindungsgemäß durch ein Verfahren zur Renaturierung von denaturierten Proteinen in Lösung in einem Renaturierungspuffer, welches dadurch gekennzeichnet ist, daß man eine Lösung des zu renaturierenden Proteins in der Konzentration, bei der im gewählten Puffer die höchste Ausbeute an nativem renaturierten Protein erhalten wird (kritische Konzentration), herstellt und nach Bildung des Faltungsintermediats weiteres zu renaturierendes Protein in der zur Erzielung erforderlichen Menge zusetzt.

Das Verfahren beruht auf der Entdeckung, daß eine möglichst hohe Ausbeute an renaturiertem Protein bei gleichzeitig maximaler Geschwindigkeit der Renaturierung dann erzielt werden kann, wenn ein bestimmter kritischer Konzentrationswert des denaturierten, zu renaturierenden Proteins in der Lösung nicht überschritten wird, wobei dieser Wert von der Art des jeweiligen Proteins und den Bedingungen, unter denen die Renaturierung durchgeführt wird, abhängt. Hierbei kommt es darauf an, daß das Verhältnis von zwei miteinander konkurrierenden Reaktionen, nämlich die unerwünschte Bildung von inaktivem Protein (z. B. Aggregaten) einerseits und die Bildung eines Faltungsintermediats andererseits, welches schließlich ins endgültige native renaturierte Protein übergeht, so verschoben wird, daß nur ein möglichst geringer Anteil an inaktivem Protein auftreten kann. Hierzu ist es erforderlich, die proteinspezifische und bedingungsspezifische kritische Konzentration des zu renaturierenden Proteins, die Renaturierungskinetik und die Zeit bis zur Bildung des Faltungsintermediats zu berücksichtigen.

Diese drei für das Verfahren wesentlichen Parameter lassen sich jeweils ohne weiteres bestimmen. Die kritische Konzentration läß sich durch eine Eichkurve feststellen, die man erhält, indem man im jeweils gewählten Renaturierungspuffer verschiedene Konzentrationen an denaturiertem Protein einsetzt und die dabei in einem vorgegebenen Zeitraum erzielte Endausbeute an renaturiertem Protein mißt. Die kritische Konzentration entspricht dem Wert, bei dem die höchste Ausbeute (relative Ausbeute in % der Ausgangskonzentration) an nativem renaturiertem Protein erhalten wird.

Die Renaturierungskinetik kann ebenfalls nach bekannten Methoden bestimmt werden. Dabei wird bei der maximal moglichen Konzentration die für die Umwandlung ins renaturierte Protein erforderliche Zeit bestimmt.

Die Zeit bis zur Bildung des Faltungsintermediats läßt sich schließlich nach einer ganzen Reihe von verschiedenen Verfahren bestimmen. Eine erste Bestimmungsmethode besteht in der Messung des Zirkulardichroismus (CD). Hierbei wird zweckmäßig eine Wellenlänge ausgewählt, bei der ein großes Signal erhalten wird und dann die entsprechende Kurve gemessen, wobei in der Regel ein steiler Anstieg in relativ kurzer Zeit erfolgt und die Kurve dann abflacht, sobald das Faltungsintermediat gebildet ist. Eine weitere Möglichkeit besteht darin, das Verschwinden der SH-Gruppen durch Bildung intramolekularer Disulfidbrücken zu verfolgen. Hierbei ist jedoch zu beachten, daß keine intermolekularen Disulfidbrücken gebildet werden dürfen, was durch Kontrolle des Molekulargewichts geschehen kann, welches im letzteren Falle ansteigen würde.

Eine weitere Möglichkeit besteht in der Fluoreszenzmessung. Sie beruht auf der Tryptophan- bzw. Tyrosinfluoreszenz, welche sich verändert, wenn die aromatischen Reste in eine hydrophobe Region des gefalteten Proteins überführt werden. Weitere Möglichkeiten sind limitierte Proteolyse, da mit zunehmender Bildung des Faltungsintermediats auch der proteolytische Angriff verringert wird. Ebenfalls ist es möglich, diese Bestimmung durch hydrodynamische Messungen durchzuführten. Beispielsweise wird hier die Intrinsic-Viskosität gemessen, die absinkt, da das Faltungsintermediat zur Bildung einer Lösung mit geringerer Viskosität führt.

Für die Praxis genügt es, die kritische Konzentration und die Zeit bis zur Bildung des gegen die Nebenreaktion zu inaktivem Protein geschützten Faltungsintermediats zu bestimmen und mit der kritischen Konzentration jeweils zu beginnen und nach Ablauf der Bildung des Faltungsintermediats erneut das in der Zwischenzeit aus der Lösung durch Umwandlung verbrauchte denaturierte Protein aufzustocken. Dies kann schrittweise geschehen, indem man die Zeiten und kritischen Konzentrationen vorgibt und dann jeweils nach Ablauf der Intermediatbildung die bekannte Menge an hierbei umgewandeltem denaturiertem Protein ersetzt. Alternativ läßt sich das Verfahren auch kontinuierlich gestalten, indem immer gerade so viel denaturiertes Protein neu zugegeben wird, wie durch Neubildung von Faltungsintermediat abgeht. Auf diese Weise läßt sich die Renaturierung in einem Kreisverfahren durchführen, bei welchem man beispielsweise das zu renaturierende Protein in einem dafür geeigneten Lösungsmittel, wie z. B. stark verdünnter Salzsäure, gelöst oder in lyophilisierter Form in jeweils solcher Menge kontinuierlich dem Renaturierungsreaktor zuführt, daß die optimale Renaturierungskonzentration erhalten wird. Der Renaturierungspuffer wird gleichzeitig in solcher Zusammensetzung kontinuierlich zugeführt, daß die Bedingungen hinsichtlich pH-Wert, Substanzkonzentrationen und dergleichen konstant bleiben. Durch entsprechende Gestaltung des Reaktors, der bespielsweise schleifenförmig aufgebaut sein kann und die durch die Zugabegeschwindigkeit und -menge von Protein und Puffer definierte Strömungsgeschwindigkeit kann eine Aufenthaltsdauer festgelegt werden, die der Zeit bis zur Bildung des Faltungsintermediats entspricht. Danach wird reaktiviertes Protein kontinuierlich abgezogen, beispielsweise durch Abtrennung mittels Ultrafiltration, umgekehrte Osmose und dergleichen und als Retentat gegebenenfalls einer Auftrennung in inaktive Aggregate und aktives Protein unterzogen. Bevorzugt wird das kontinuierliche Verfahren so durchgeführt, daß die abgetrennte Pufferlösung dem Reaktor kontinuierlich wieder zugeführt wird.

Bei der kontinuierlichen Durchführung des erfindungsgemäßen Verfahrens ist es bevorzugt, anfangs wie beim nichtkontinuierlichen Verfahren das denaturierte Protein in der vorstehend beschriebenen Weise so lange zuzudosieren, bis sich eine optimale Konzentration an renaturiertem nativem Protein eingestellt hat. Sobald dies erreicht ist, wird mit der kontinuierlichen Entnahme der relativ konzentrierten Lösung an reaktiviertem Protein begonnen und dann erst ins wirklich voll kontinuierliche Verfahren übergegangen. Die Eintrittsphase, in der die Konzentration an reaktiviertem Protein noch ansteigt, ist daher der eigentlichen kontinuierlichen Durchführung vorgeschaltet. Als optimale Konzentration von renaturiertem Protein wird dabei die höchstmögliche Konzentration verstanden, bei der noch keine Probleme mit Unlöslichwerden von Bestandteilen des Reaktionssystems auftreten.

Vorzugsweise wird beim erfindungsgemäßen Verfahren lyophilisiertes denaturiertes Protein eingesetzt. Handelt es sich jedoch um ein Protein, das nur langsam und schwierig in Lösung zu bringen ist, so erfolgt die Zugabe in gelöster Form, um das Zeitmerkmal des erfindungsgemäßen Verfahrens definiert einhalten zu können.

Durch das erfindungsgemäße Verfahren wird zwar die unerwünschte und mit der Renaturierung konkurrierende Aggregatbildung minimalisiert, aber nicht völlig unterdrückt. Die gebildeten inaktiven Aggregate weisen jedoch ein wesentlich höheres Molekulargewicht auf und lassen sich daher leicht vom renaturierten Protein durch Trennungsmethoden entfernen, welche diesen Molekulargewichtsunterschied ausnutzen, beispielsweise also durch Molekularsiebfraktionierung, gegebenenfalls Ultrafiltration mit geeigneten Ausschlußgrenzen, Gradienten-Zentrifugation und dergleichen. So abgetrennte Aggregate können dann nach ihrer Solubilisierung wieder in das Renaturierungsverfahren eingesetzt werden.

Als Renaturierungspuffer kommen die hierfür bekannten Zusammensetzungen in Betracht. Der pH-Wert muß im Rahmen des pH-Wertes liegen, bei dem das betreffende Protein gute Stabilität aufweist, zweckmäßig nahe beim pH-Wert der maximalen Stabilität liegen. Um den denaturierenden Einfluß von Verunreinigungen, beispielsweise von Schwermetallen, auszuschließen, wird zweckmäßig in Gegenwart von Komplexbildnern, wie EDTA und ähnlichen Verbindungen gearbeitet. So ist beispielsweise für den Fall der Lactat-Dehydrogenase Phosphatpuffer pH 6 bis 8, der ungefähr 1 mM EDTA und 1 mM DTE enthält, brauchbar. Für Lysozym hat sich schwach alkalischer Tris/HCl-Puffer bewährt, der neben EDTA eine SH-Gruppen-haltige Substanz und eine kleine Menge des korrespondierenden Disulfids enthält. Für die Renaturierung von tPA eignet sich bespielsweise der in der Deutschen Patentanmeldung P 3 537 708 beschriebene Reaktivierungspuffer. Für zahlreiche weitere Proteine sind geeignete Renaturierungspuffer bekannt oder lassen sich auf Basis der bekannten Eigenschaften des Proteins, insbesondere hinsichtlich der pH-Stabilität, Gehalt an SH-Gruppen, Schwermetallempfindlichkeit und dergleichen vom Fachmann anhand einiger Vorversuche herausfinden.

Bekannt war es, daß für die Renaturierung bestimmte Konzentrationsgrenzwerte nicht überschritten werden sollten. Neu ist die Erkenntnis, daß größere Mengen an denaturiertem Protein nicht etwa größere Mengen an Lösungsvolumen erfordern, um größere Mengen an renaturiertem Protein zu erzielen, sondern daß man in derselben Lösung durch ständige Neinzugabe oder intermittierende Neuzugabe von denaturiertem Protein weiterarbeiten kann und damit zu wesentlich höheren Konzentrationen an renaturiertem Protein gelangen kann, als dies nach dem Stand der Technik für möglich gehalten wurde.

Die folgenden Beispiele erläutern die Erfindung weiter.

### Beispiel 1

### Pulsreaktivierung von Lysozym

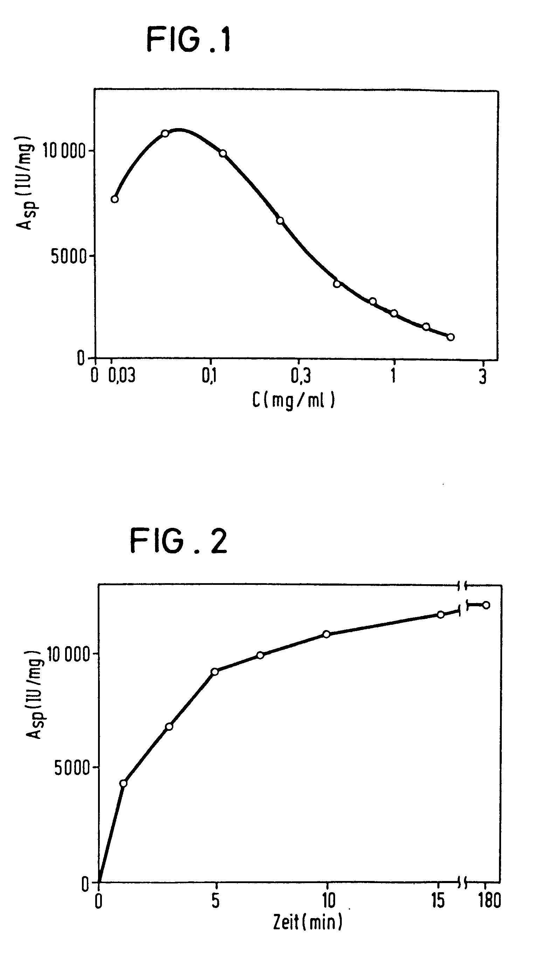

a) Vorbereitung der Enzymlösungen
   Konzentrationsbestimmung (A: Absorption einer 1%igen Lösung bei 280 nm):
   - natives Lysozym:: A = 26,3
   - reduziertes Lysozym:: A = 23,7
   Bestimmung der spezifischen Aktivität:
   - 2mg Lysozym in 1 ml 0,066 mol/l K-Phosphat, pH 6,2 lösen; Konzentration bestimmen durch UV Absorption
   - Testlösung: 2,5 mg Zellwand von Micrococcus lysodeikticus in 10 ml 0,066 mol/l K-Phosphat, pH 6,2 suspendieren
   - Test bei 25°C: 1 ml Zellwandsuspension plus 0,03 ml Lysozymlösung (1:40 vorverdünnt); Abnahme der Trübung bei 450 nm verfolgen
      Spez. Aktivität = 60 000 IU/mg
      Reduktion/Denaturierung:
   - 500 mg Lysozym 3 h bei 25°C inkubieren in 0,1 mol/1 Tris/HCL, pH 8,6 + 6 mol/l Gdn·HCl + 150 mmol/l DTE
   - pH auf pH 3 einstellen und Probe teilen
      Teil A
   - Gelfiltration über Sephadex G 25 F (Säulenvolumen = 270 ml) in 0,01 mol/l HCl
   - Peakfraktionen vereinigt und lyophilisiert
      Teil B
   - Dialyse gegen 3 x 1 L 0,01 mol/l HCl bei 4°C
   - Lyophilisation
      Beide Verfahren der Entsalzung (Teil A und B) ergeben reduziertes Lysozym, das sich bezüglich der Reaktivierungseigenschaften nicht unterscheidet.
   - Das lyophilisierte, reduzierte Enzym wird bei -40°C unter N₂ aufbewahrt.
      Reoxidation/Reaktivierung:
   - 12 mg reduziertes Lysozym in 1 ml 0,01 mol/l HCl lösen
   - Milliporefiltration
   - Konzentrationsbestimmung durch UV-Absorption
   - Reoxidation bei 25°C durch Verdünnen in 0,1 mol/l Tris/HCl, pH 8,2 + 1 mmol/l EDTA + 3 mmol/l GSH + 0,3 mmol/l GSSG
b) Bestimmung der kritischen Konzentration der Renaturierung
   - Reduktion/Reoxidation wie oben beschrieben erfolgt durch Variation der Proteinkonzentration im Reaktivierungsansatz. Das Ergebnis zeigt Fig. 1, wo die Konzentrationsabhängigkeit der Reaktivierung von reduziertem Lysozym nach 20 h Reoxidation bei 25°C graphisch dargestellt ist.
c) Zeit bis zur Bildung des Faltungsintermediats
   - Durch Reduktion/Reoxidation wie oben beschrieben wird die Kinetik der Reaktivierung von reduziertem Lysozym bei einer Proteinkonzentration von 0,03 mg/ml; 25°C bestimmt. Das Ergebnis ist in Fig. 2 graphisch dargestellt.
d) "Puls-Reaktivierung" von reduziertem Lysozym
   - 1,6 bzw. 2,4 ml 0,125 mol/l Tris/HCl, pH 8,2 + 1,25 mmol/l EDTA + 3,75 mmol/l GSH + 0,375 mmol/l GSSG vorgelegt.
      1. Versuch
   - In Zeitabständen t = 10 min 20 x jew. 20 µl reduziertes Lysozym in 0,01 mol/l HCl zugeben. Konzentrationserhöhung pro Puls: 0,11 - 0,13 mg/ml Endkonzentration nach 20 Pulsen: 2,15 mg/ml
   - Vergleichsreaktivierung ohne Puls: Einmalige Zugabe auf gleiche Endkonzentration. Die Pufferbedingungen entsprechen nach Verdünnung dem o. g. Reoxidationspuffer.
   - Nach 2 h Reaktivierung bei 25°C
      - Pulsreaktivierung:: 2200 IU/mg
      - Vergleich ohne Puls:: 470 IU/mg
      2. Versuch:
   - In Zeitabständen t = 10 min 20 x jew. 30µl reduziertes Lysozym in 0,01 mol/l HCl zugeben. Konzentrationserhöhung pro Puls: 0,034-0,042 mg/ml Endkonzentration nach 20 Pulsen: 0,68 mg/ml
   - Vergleichsaktivierung: Einmalige Zugabe auf gleiche Endkonzentration
   - Nach 0,5 h Reaktivierung bei 25°C:
      - Pulsreaktivierung:: 7660 IU/mg
      - Vergleich ohne Puls:: 1400 IU/mg
      3. Versuch
   - In Zeitabständen t = 20 min 20 x jew. 20 µl reduziertes Lysozym in 0,01 mol/l HCl zugeben. Konzentrationserhöhung pro Puls: 0.035-0,044 mg/ml Endkonzentration nach 20 Pulsen: 0,71 mg/ml
   - Vergleichsreaktivierung: Einmalige Zugabe auf gleiche Endkonzentration
   - Nach 24 h Reaktivierung bei 25°C
      - Pulsreaktivierung:: 10100 IU/mg
      - Vergleich ohne Puls:: 1400 IU/mg
   Aus diesen Resultaten ergibt sich, daß
   - Die Reaktivierungsausbeute bei Proteinkonzentrationen >0,1 mg/ml kontinuierlich abnimmt.
   - Bei 25°C ist die Reaktivierung unter den gegebenen Reoxidationsbedingungen nach 15 min fast vollständig abgeschlossen (Fig. 2)
   - Durch "Puls-Reaktivierung" gemäß Erfindung kann im Verleich zur ungepulsten Reaktivierung eine 5 bis 7fach höhere Ausbeute erreicht werden.

### Beispiel 2

### "Puls-Reaktivierung" von LDH-M₄

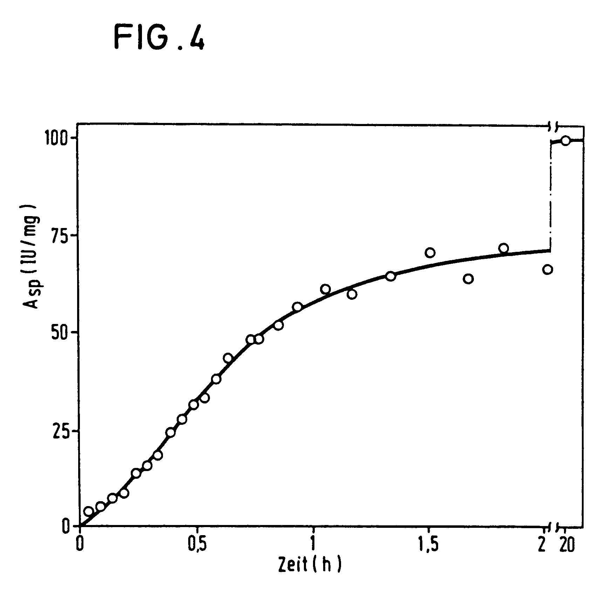

a) Vorbereitung der Enzymlösung:
   - LDH-M₄ (aus Schweinemuskel) Suspension in (NH₄)₂SO₄ (Hersteller: Boehringer Mannheim GmbH, Best.Nr. 107077) wird zentrifugiert und das Pellet mit 1/6 des ursprünglichen Volumens in 0,1 mol/l K-Phosphat, pH 7,6 + 1 mmol/l EDTA + 1 mmol/l DTE aufgenommen.
   - und gegen 2 x 1 L des gleichen Puffers bei 4°C dialysiert.
   - Konzentrationsbestimmung über A = 14 (A = Absorption einer 1%igen Lösung bei 280 nm)
   - Der Aktivitätstest bei 25°C entsprechend R. Hermann, R. Jaenicke & R. Rudolph, Biochemistry 20 (1981) 5195-5201 ergibt
      Spez. Aktivität = 300 IU/mg
      Denaturierung/Reaktivierung
   - Denaturierung: 1:5 Verdünnung mit 0,1 mol/1 H₃PO₄ 6 Min. Inkubation bei 0°C
   - Reaktivierung 1:3 Verdünnung mit 0,1 mol/l K-Phosphat, pH 12,65 + 1 mmol/1 EDTA + 1 mmol/l DTE bei 20°C; nach der Mischung war der pH der Lösung pH 7,6
b) Bestimmung der kritischen Konzentration der Renaturierung
   - Denaturierung/Reaktivierung erfolgen wie oben angegeben unter Variation der Proteinkonzentration im Denaturierungsansatz.

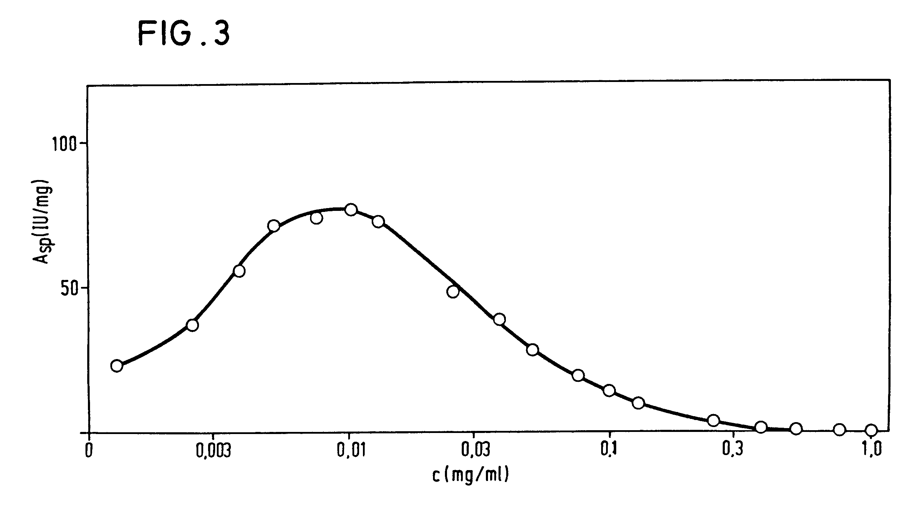

      Fig. 3 zeigt die erhaltene Konzentrationsabhängigkeit der Reaktivierung von LDH-M₄ nach Säuredenaturierung, wobei die Aktivität nach 20 h Reaktivierung bei 25°C gegen die Konzentration aufgetragen ist.
c) Bestimmung der Zeit bis zur Bildung des Faltungsintermediats
   - Durch Denaturierung/Renaturierung wie oben wird die Kinetik der Reaktivierung von säuredenaturierter LDH-M₄ bei einer Proteinkonzentration von 0,01 mg/ml; 25°C bestimmt. Das Ergebnis zeigt Fig. 4
d) Kontinuierliche Reaktivierung von säuredenaturierter LDH-M₄.
   In einem geschlossenen Reaktor wurden kontinuierlich säuredenaturierte LDH-M₄ und Reaktivierungspuffer eingespeist.

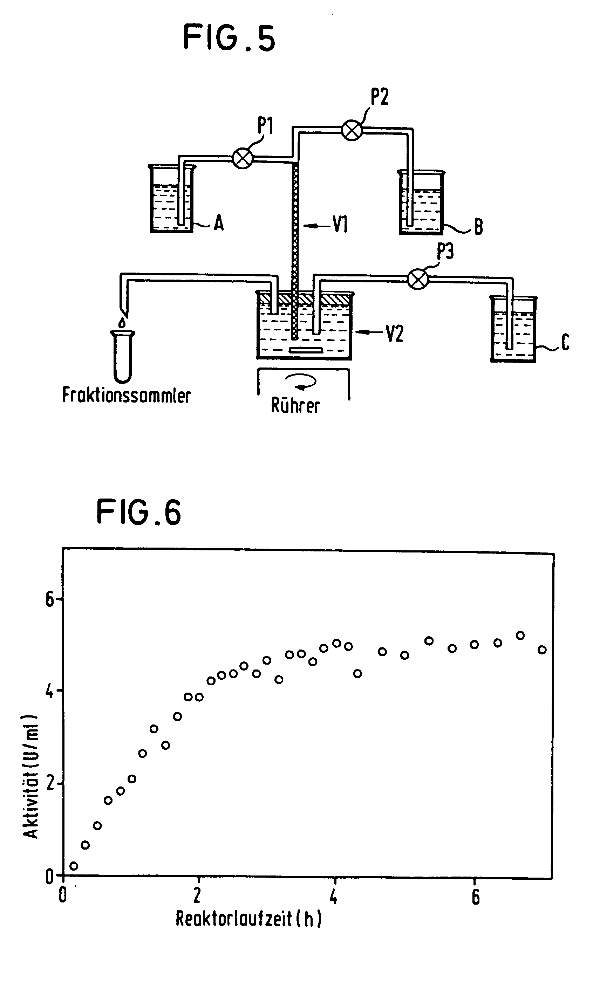

Der verwendete Reaktivierungsreaktor ist schematisch in Figur 5 dargestellt.

Aus Vorratsgefäßen A und B wird kontinuierlich Lösung des Enzyms und Denaturierungslösung über die Pumpen P₁ und P₂ zugeführt und in der Mischstrecke V1 denaturiert. Die denaturierte Enzymlösung wird in den Renaturierungsreaktor V2 kontinuierlich eingeführt. Gleichzeitig wird Renaturierungslösung aus dem Vorratsgefäß C über die Pumpe P₃ ebenfalls dem Reaktor V2 kontinuierlich zugeführt. Renaturierte Lösung wird kontinuierlich abgezogen und im Fraktionssammler gesammelt. Die Zusammensetzung der Lösungen und die im Reaktor vorliegenden Bedingungen waren wie folgt:
A: 7,43 mg/ml LDH-M₄ in 0,1 mol/1 K-Phosphat, pH 7,6 + 1 mmol/l DTE + 1 mmol/l EDTA bei 0°C
B: Denaturierungspuffer: 0,1 mol/l H₃PO₄ bei 0°C
C: Renaturierungspuffer: 0,1 mol/l K-Phosphat, pH 12,6 + 1 mmol/l DTE + 1 mmol/l EDTA bei 25°C (pH nach Mischung mit B: pH 7,6)
V1: Denaturierungsvolumen V₁ = 0,25 ml. Ergibt eine Denaturierungsdauer von 3 min. bei 0°C
V2: Renaturierungsvolumen V₂ = 58 ml; 25°C
   - Pumpengeschwindigkeit:: P₁: 1 ml/h
   P₂: 4 ml/h
   P₃: 10 ml/h

Bei der gegebenen Reaktorgröße/Pumprate ergibt sich für die mittlere Verweilzeit eines Moleküls im Reaktor 2,9 Stunden.
Bei dem Lauf wurden Proben a 2,5 ml gesammelt und die Endreaktivierung nach 24 h bei 25°C bestimmt.

Fig. 6 zeigt den Aktivitätsanstieg bei Aufladen des Reaktors.

Nach etwa 4 h wird eine konstante Reaktivierungsausbeute erreicht.

| | |
|---|---|
| - Endkonzentration der Reaktivierung: | 0,5 mg/ml |
| - Ausbeute: Kontinuierliche Reaktivierung: | 10,2 IU/mg |
| - Vergleich ohne Puls: | 1,9 IU/mg |

Die obigen Ergebnisse zeigen, daß erfindungsgemäß eine etwa 5,4fach höhere Ausbeute erreicht wird, als bei Direktreaktivierung bei gleicher Endkonzentration.

### Beispiel 3

### Puls-Reaktivierung von rec-tPA

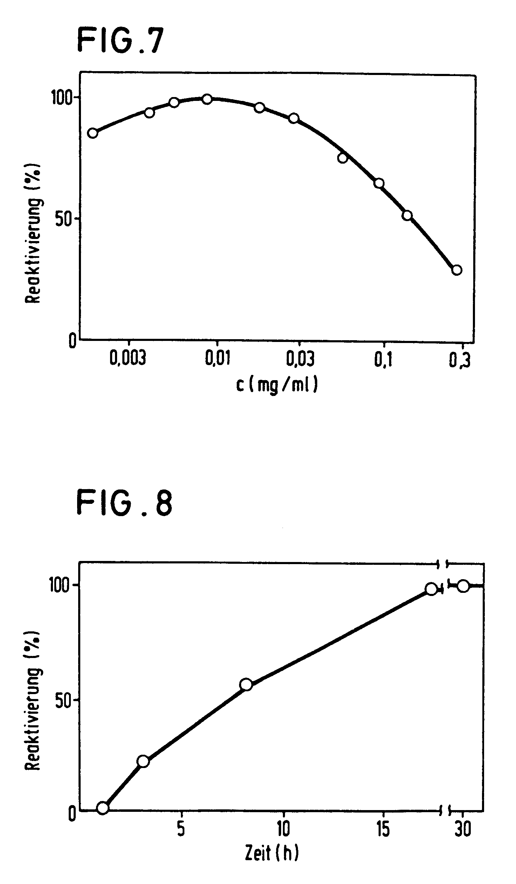

a) Vorbereitung der "refractile bodies (RB)"
"RB"-Präparation durch Zellaufschluß und diverse Zentrifugations-/Waschschritte erfolgte analog dem Verfahren der EP-A1-0114 506, Beispiel 7. tPA ist in den "RB's" mit Fremdprotein verunreinigt, inaktiv und unlöslich.
Solubilisierung durch Reduktion/Denaturierung:
- "RB's" aus 2,5 g Zellen 3 h bei 25°C inkubiert in 10 ml 0,1 mol/l Tris/HCL, pH 8,6 + 6 mol/l Gdn-HCL + 1 mmol/l EDTA + 150 mmol/DTE
- Gesamtprotein in den "RB's" nach Reduktion: 41,5 mg
pH auf pH 3 einstellen mit HCl konz. und Gelfiltration über Sephadex G25 F (Säulenvolumen = 270 ml) in 0,01 mol/l HCL.
b) Bestimmung der Konzentrationsabhängigkeit der Reaktivierung
Die saure Fraktion des reduzierten Proteins wird mit maximaler Reaktivierbarkeit (c = 0,83 mg/ml) reoxidiert durch 1:3 bis 1:500 Verdünnung in 0,1 mol/l Tris/HCL, pH 10,4 + 1 mmol/l EDTA + 0,5 mol/l L-Arginin + 1 mg/ml BSA + 1 mmol/l GSH + 0,2 mmol/l GSSG.
Die Reoxidationsbedingungen bei verschiedenen Verdünnungen werden konstant einjustiert. Nach 25-27 h Reoxidation bei 25°C wird die Aktivität bestimmt. Vor der Aktivierungsmessung wird mit Reoxidationspuffer auf gleiche Endkonzentration eingestellt. Die Ergebnisse zeigen Tabelle 1 und Figur 7 der Zeichnung.

**Tabelle 1**

| Konzentrationsabhängikeit der Reaktivierung von rec-tPA | | |
|---|---|---|
| Protein konzentration¹ (µg/ml) | Ausbeute² | Stimulierbarkeit (Faktor) |
| 277 | 31.3 | 16,8 |
| 138 | 52,4 | 17,0 |
| 92,2 | 64,9 | 18,5 |
| 55,3 | 74,9 | 21,5 |
| 27,7 | 91,5 | 17,5 |
| 17,3 | 95,6 | 25,3 |
| 8,68 | 100,0 | 20,5 |
| 5,53 | 97,8 | 19,8 |
| 3,95 | 94,4 | 20,6 |
| 1,84 | 85,3 | 16,7 |

| | | |
|---|---|---|
| ¹ Proteinkonzentration bei Reaktivierung (Gesamtprotein) | | |
| ² Bezogen auf maximalen Reaktivierungswert | | |

c) Bestimmung der Kinetik der Reaktivierung
- Reduktion/Reoxidation werden wie oben durchgeführt.
   Aufgrund der Unreinheit des Materials konnte die Zeit bis zur Bildung des Faltungsintermediats nicht direkt gemessen werden, sondern wurde indirekt über die Kinetik der Reaktivierung und Variation der Pulsrate zu etwa 40 bis 60 Minuten bestimmt.
   Figur 8 zeigt die Kinetik der Reaktivierung von rec-tPA bei einer Gesamtproteinkonzentration von 15 µg/ml bei 20°C. Reaktivierung bezogen auf Endausbeute nach 30 h.

d) "Puls-Reaktivierung" von rec-tPA
In Zeitintervallen zwischen 2 min und 45 min wurde 15 x jeweils 10 µl reduzierter rec-tPA in 0,01 mol/l HCl zu 300 µl Reaktivierungspuffer gegeben (25°C).
- Reaktivierungspuffer vor Pulsreaktivierung: 0,15 mol/l Tris/HCl, pH 10,5 + 1,5 mmol/l EDTA + 0,5 mol/l L-Arginin + 0,3 mmol/l GSSG + 1,5 mmol/l GSH
- Reaktivierungspuffer nach Zugabe des reduzierten Proteins:
0,1 mol/l Tris/HCl, pH 10,4 + 1 mmol/l EDTA + 0,33 mol/l L-Arginin + 0,2 mmol/l GSSG + 1 mmol/l GSH.
- Konzentrationserhöhung pro Puls: 18 - 27 µg/ml
- Endkonzentration nach 15 Pulsen: 227 µg/ml
Die Aktivitätsmessung erfolgt nach 24 h Reoxidation bei 20°C. Die Ergebnisse zeigt Tabelle 2:

**Tabelle 2**

| Puls-Reaktivierung von rec-tPA | | |
|---|---|---|
| Pulsrate (min) | Ausbeute¹ (%) | Stimulierbarkeit (Faktor) |
| 2 | 31,3 | 7,1 |
| 10 | 38,6 | 8,1 |
| 20 | 39,5 | 9,0 |
| 30 | 44,8 | 8,2 |
| 45 | 45,8 | 7,8 |

| | | |
|---|---|---|
| ¹ Bezogen auf maximale Reaktivierung entsprechend Fig. 7 | | |

Kommentar:
- Bei dem verwendeten Ausgangsmaterial nimmt die Reaktivierungsausbeute - bezogen auf eingesetzte Proteinmenge - bei Konzentrationen > 20 µg/ml ab (Figur 7). Die Stimulierbarkeit durch CNBr-FSP erweist sich im Rahmen der Meßgenauigkeit als unabhängig von der Reaktivierungskonzentration (Tabelle 1).
- Bei 20°C beträgt die Halbwertszeit der Reaktivierung unter den gegebenen Versuchbedingungen 7± 2 Stunden.
- Die Reaktivierungsausbeute steigt mit zunehmender Verweilzeit zwischen den Reaktivierungspulsen (Tabelle 2).

## Patentansprüche

1. Verfahren zur Renaturierung von denaturierten Proteinen in Lösung in einem Renaturierungspuffer,
**dadurch gekennzeichnet,**
daß man eine Lösung des zu renaturierenden Proteins in der Konzentration, bei der im gewählten Puffer die höchste Ausbeute an nativem renaturierten Protein erhalten wird (kritische Konzentration), herstellt und nach Bildung des Faltungsintermediats weiteres zu renaturierendes Protein in der zur Erzielung erforderlichen Menge zusetzt.

2. Verfahren nach Anspruch 1, **dadurch gekennzeichnet,** daß das Verfahren diskontinuierlich durchgeführt wird, indem jeweils nach Ablauf der zur Bildung des Faltungsintermediats erforderlichen Zeit, die zur Erzielung der kritischen Konzentration erforderliche Menge an denaturiertem Protein zugesetzt wird.

3. Verfahren nach Anspruch 1, **dadurch gekennzeichnet,** daß zur kontinuierlichen Renaturierung einem Reaktor kontinuierlich die zur Erzielung der kritischen Konzentration erforderliche Menge an denaturiertem Protein und die entsprechende Menge an Renaturierungspuffer zugeführt werden und die Verweildauer der Mischung im Reaktor auf die zur Bildung des Faltungsintermediats benötigte Dauer eingeregelt wird.

4. Verfahren nach Anspruch 3,
**dadurch gekennzeichnet,**
daß das denaturierte Protein dem Reaktor so lange zugesetzt wird, bis sich die höchstmögliche Konzentration an renaturiertem Protein, bei der noch keine Bestandteile des Reaktionssystems unlöslich werden (optimale Konzentration) eingestellt hat und dann die so erhalten Lösung kontinuierlich entnommen und durch Lösung von denaturiertem Protein in optimaler Konzentration entsprechend ersetzt wird.

5. Verfahren nach einem der vorhergehenden Ansprüche,
**dadurch gekennzeichnet,**
daß gebildete inaktive Proteinaggregate auf Basis ihres höheren Molekulargewichts vom renaturierten Protein abgetrennt und nach Auflösung wieder in das Verfahren eingesetzt werden.

6. Verfahren nach einem der vorhergehenden Ansprüche,
**dadurch gekennzeichnet,**
daß das denaturierte Protein in lyophilisierter oder in vorgelöster Form in das Verfahren eingesetzt wird.

## Claims

1. Process for the renaturation of denatured proteins in solution in a renaturation buffer, characterised in that one prepares a solution of the protein to be renatured in the concentration in which, in the selected buffer, the highest yield of native renatured protein is obtained (critical concentration) and, after formation of the folding intermediate, adds further protein to be renatured in the amount necessary for the achievement.

2. Process according to claim 1, characterised in that the process is carried out discontinuously in that, in each case after expiry of the time necessary for the formation of the folding intermediate, the amount of denatured protein necessary for the achievement of the critical concentration is added thereto.

3. Process according to claim 1, characterised in that, for the continuous renaturation, into a reactor are continuously introduced the amount of denatured protein for the achievement of the critical concentration and the corresponding amount of renaturation buffer and the residence time of the mixture in the reactor is adjusted to the period needed for the formation of the folding intermediate.

4. Process according to claim 3, characterised in that the denatured protein is left in the reactor until the highest possible concentration of renatured protein is reached at which still no components of the reaction system become insoluble (optimum concentration) and then the so obtained solution is removed continuously and correspondingly replaced by a solution of denatured protein in optimum concentration.

5. Process according to one of the preceding claims, characterised in that inactive protein aggregate formed is separated off on the basis of its higher molecular weight from renatured protein and, after dissolving, again introduced into the process.

6. Process according to one of the preceding claims, characterised in that the denatured protein is introduced into the process in lyophilised or pre-dissolved form.

## Revendications

1. Procédé de renaturation et de dénaturation de protéines en solution dans un tampon de renaturation, caractérisé en ce que l'on prépare une solution de la protéine à renaturer à la concentration à laquelle on obtient, dans le tampon choisi, le rendement le plus élevé en protéine native renaturée (concentration critique), et, après formation de l'intermédiaire de repliement, on ajoute un supplément de protéine à renaturer en la quantité nécessaire pour l'obtention.

2. Procédé selon la revendication 1, caractérisé en ce que le procédé est mis en oeuvre de manière discontinue en ajoutant la quantité de protéine dénaturée nécessaire pour obtenir la concentration critique lorsque le temps nécessaire à la formation de l'intermédiaire de repliement s'est écoulé.

3. Procédé selon la revendication 1, caractérisé en ce que, pour la renaturation continue, on introduit en continu dans un réacteur la quantité nécessaire de protéine dénaturée pour obtenir la concentration critique et la quantité correspondante de tampon de renaturation et on règle le temps de séjour du mélange dans le réacteur en fonction de la durée nécessaire pour la formation de l'intermédiaire de repliement.

4. Procédé selon la revendication 3, caractérisé en ce que l'on ajoute la protéine dénaturée au réacteur jusqu'à l'établissement de la concentration la plus élevée possible de protéine renaturée à laquelle aucun composant du système réactionnel ne devient encore insoluble (concentration optimale) puis on prélève en continu la solution ainsi obtenue et on la remplace de manière correspondante par une solution de protéine dénaturée de concentration optimale.

5. Procédé selon l'une des revendications précédentes, caractérisé en ce que les agrégats protéiques inactifs formés sont séparés de la protéine renaturée grâce à leur poids moléculaire plus élevé et sont de nouveau utilisés dans le procédé après dissolution.

6. Procédé selon l'une des revendications précédentes, caractérisé en ce que la protéine dénaturée est utilisée dans le procédé sous forme lyophilisée ou dissoute au préalable.
